(19) Europäisches Patentamt
European Patent Office
Office européen des brevets

(11) Publication number: 0 336 193 A2

# (12) EUROPEAN PATENT APPLICATION

(21) Application number: 89104965.2

(22) Date of filing: 20.03.89

(51) Int. Cl.4: A61K 7/043

(30) Priority: 08.04.88 IT 2013088

(43) Date of publication of application:
11.10.89 Bulletin 89/41

(84) Designated Contracting States:
AT BE CH DE ES FR GB GR IT LI LU NL SE

(71) Applicant: ENICHEM SYNTHESIS S.p.A.
Via Ruggero Settimo 55
I-90139 Palermo(IT)

(72) Inventor: Traverso, Enrico
Via Monte Bianco, 22
Monza (Milano)(IT)

(74) Representative: Cioni, Carlo et al
c/o ENIRICERCHE S.p.A. BRELID Via F. Maritano 26
I-20097 San Donato Milanese(IT)

## (54) Photocromic nail enamel or varnish and the process for the preparation thereof.

(57) Photochromic nail enamel and varnish compositions are described which are characterized in that they contain a photochromically effective amount of at least one compound of general formula (I)

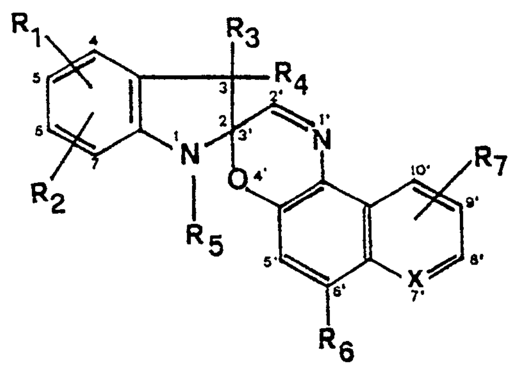

e.g. 1,3,3,4,5- or 1,3,3,5,6-pentamethyl-spiro[indoline-2,3′-[3H]-naphtho[2,1-b][1,4]oxazine], 1,3,3-trimethyl-spiro[indoline-2,3′-[3H]naphtho[2,1-b][1,4]oxazine], 1,3,3,4,5- or 1,3,3,5,6-pentamethyl-spiro[indoline-6′-(1-piperidyl)-2,3′-[3H]naphtho[2,1-b][1,4]oxaxine], 1,3,3-trimethyl-spiro[indolien-6′-(1-piperidyl) -2,3′-[3H]-naphtho[2,1-b][1,4]oxazine], 1,3,3-trimethyl-spiro[indoline-6′-(1-morpholyl)-2,3′-[3H]naphtho[2,1-b] [1,4]-oxazine], and 1,3,3-trimethyl-spiro[indoline-6′-(1-piperidyl)-9′-methoxy-2,3′-[3H]naphtho[2,1-b][1,4] oxazine].

EP 0 336 193 A2

## PHOTOCHROMIC NAIL ENAMEL OR VARNISH AND THE PROCESS FOR THE PREPARATION THEREOF

The present invention refers to a photochromic nail enamel or varnish and to the process for the preparation thereof.

For the sake of clarity, the term "enamel" identifies a pigmented varnishing product which resembles ceramic enamels, while the term "varnish", according to a definition that is commonly accepted in this field, is used to represent a varnishing transparent product which may or may not be pigmented.

The term "photochromic" before "nail enamel or varnish" means that the nail enamel or varnish of the present invention, upon absorption of electromagnetic energy, rapidly changes in color, returning to the original one if the radiation is discontinued.

Photochromic nail lacquers obtained by the addition of photochromic spiro-[2H]-1-benzopyran-2,2′-indolines to conventional nail lacquer compositions are known in the patent literature (see Japanese patent application publication (kokai) no. 50 52,245 (Derwent 77707W) -Chem. Abstr. 83, 136729r; and Japanese patent application publication (kokai) no. 56 100,709 (Derwent 70770D) - Chem. Abstr. 96, 11514d).

Said spiro-benzopyrano-indolines, however, have a very low stability when exposed to sunlight (light fatigue), rapidly converting into non photochromic species. Furthermore, spiro-benzopyrano-indolines, in spite of their short activation time, require very long times to return to the original, inactivated form.

Therefore, the use of spiro-benzopyrano-indolines in the preparation of nail lacquers is not advisable; in fact, after a first exposure to the light source, when the radiation is discontinued, no changes in color can be observed unless about one hour later and owing to the poor stability of these compounds, very frequent applications are needed.

It has now been found that it is possible to prepare nail enamels or varnishes that :

- when exposed to sun light or to artificial light sources with an U.V. component of suitable wavelenght, rapidly change color,
- when the exposure is discontinued, rapidly return to their original color, and
- are sufficiently stable not to require more frequent applications than conventional nail enamels or varnishes,

using one or more spiro-indoline-naphthooxazines or spiro-indoline-pyridobenzooxazines to provide the composition with the desired photochromic characteristics.

More particularly, it has been found that :

- very small amounts of said spiro-indoline-naphthooxazines or -pyridobenzooxazines are sufficient to impart photochromic characteristics to the conventional nail varnish or enamel compositions;
- said compounds show very short "activation" and "inactivation" times (a few second exposure to sunlight or to another light source with an U.V. component of wavelenght between 320 and 380 nm, is sufficient to completely bring about the photochromic effect and, when the exposure to the light source is discontinued, 2-3 minutes are sufficient to return to the original color);
- their resistance to fatigue and ageing is sufficiently high; and, finally,
- on exposure to sunlight they manifest aesthetically pleasing color tones.

A first object of the present invention is therefore a nail varnish or enamel containing at least one photochromic compound of general formula (I)

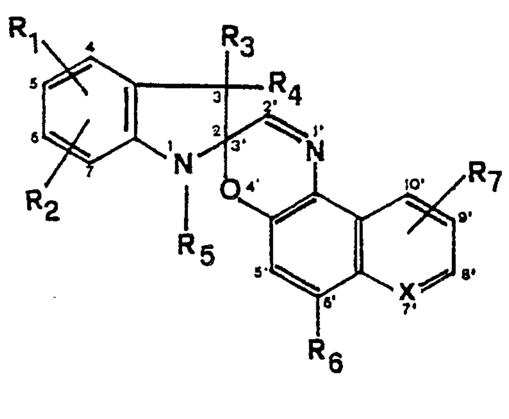

(I)

wherein

$R_1$ and $R_2$, which may be linked to any of positions 4, 5, 6 or 7 of the indoline moiety, each independently, represent hydrogen, chloro, fluoro, bromo, straight or branched, optionally per-fluorinated, $(C_1-C_5)$alkyl, straight or branched $(C_1-C_5)$alkoxy, nitro, or cyano;

$R_3$ and $R_4$, each independently, represent straight or branched $(C_1-C_5)$alkyl, phenyl or benzyl; or $R_3$ and $R_4$, taken together with the adjacent carbon atom form a $(C_5-C_8)$cycloalkyl group;

$R_5$ represents straight or branched $(C_1-C_5)$alkyl, phenyl, benzyl or allyl;

$R_6$ designates hydrogen, straight or branched $(C_1-C_5)$alkyl or $-NR_8R_9$, wherein $R_8$ is straight or branched $(C_1-C_5)$alkyl, phenyl, or benzyl, $R_9$ is hydrogen or has the same meaning as $R_8$, or $R_8$ and $R_9$ taken together with the adjacent nitrogen atom, form a 5-12 membered cyclic structure which may contain an additional heteroatom selected from oxygen and nitrogen;

$R_7$, which may be linked to any of positions 7′, 8′, 9′, or 10′, represents hydrogen, fluoro, chloro, bromo, straight or branched $(C_1-C_5)$alkyl, straight or branched $(C_1-C_5)$alkoxy, cyano, thioether, or $(C_1-C_3)$-alkoxycarbonyl,

or $R_7$ may represent a fused aromatic or heterocyclic ring; and

X is =CH- or =N-.

According to a preferred embodiment of the present invention, the nail enamel or varnish will contain at least one compound of formula (I) wherein

$R_1$ and $R_2$, each independently, represent hydrogen or methyl;

$R_3$ and $R_4$ are methyl or $R_3$ and $R_4$, taken together with the adjacent carbon atom, represent a cyclohexyl group;

$R_5$ is methyl;

$R_6$ represents hydrogen or a group $-NR_8R_9$, wherein $R_8$ and $R_9$ taken together with the adjacent nitrogen atom, form a piperidyl, morpholyl, pyrrolidyl, or hexamethyleneimino group;

$R_7$ is hydrogen; and

X is =CH-.

Examples of preferred photochromic compounds employed according to the present invention are :

1,3,3,4,5- or 1,3,3,5,6-pentamethyl-spiro[indoline-2,3′-[3H]naphtho[2,1-b][1,4]oxazine]

1,3,3-trimethyl-spiro[indoline-2,3′-[3H]naphtho[2,1-b] -[1,4]oxazine]

1,3,3-trimethyl-spiro[indoline-6′-(1-piperidyl)-2,3′-[3H]naphtho[2,1-b][1,4]oxazine]

1,3,3,4,5- or 1,3,3,5,6-pentamethyl-spiro[indoline-6′-(1-piperidyl)-2,3′-[3H]naphtho[2,1-b][1,4]oxazine]

1,3,3-trimethyl-spiro[indoline-6′-(1-morpholyl)-2,3′-[3H]naphtho[2,1-b][1,4]oxazine]

1,3,3-trimethyl-spiro[indoline-6′-(1-piperidyl)-9′-methoxy-2,3′-[3H]naphtho[2,1-b][1,4]oxazine]

The nail enamels or varnishes of the present invention are prepared by combining the photochromic compound or compounds of formula (I) as defined above, with the other suitable components of conventional nail enamel or varnish formulations, provided however that said ingredients are suitably selected among those devoid of acidic character.

The spiro-indoline-naphthooxazines and spiro-indoline-pyridobenzoxazines of formula (I), in fact, have a basic character and are easily salified by acidic substances.

Salification of the compounds of formula (I) blocks the shift between the activated and the inactivated forms and also brings about decomposition of the indoline-naphthooxazines and indoline-pyridobenzoxazines of formula (I) to compounds which may have undesirable chromatic effects.

In particular, a "base" composition for the nail enamels or varnishes of the present invention, will contain one or more film-forming materials, such as, for instance, cellulose esters, e.g. cellulose acetobutyrate, cellulose acetopropionate, and cellulose nitrate, polyacrylic and polymethacrylic esters, e.g. polyalkyl-methacrylates, polyvynylacetate, polystyrene, etc., and one or more solvent/diluents such as for instance lower alkanols, e.g. ethanol, propanol, and butanol, organic acetates, e.g. ethyl acetate, butyl acetate, and amyl acetate, methylenechloride, ethylenechloride, and propylenechloride, toluene, naphthene aromatics, etc..

Typically and preferably said "base" composition will contain also one or more plsticizers, which are incorporated therein to improve some of the enamel characteristics (to increase flexibility and ease of application), which must be compatible with the film-forming materials employed and, as pointed out above, must not have acidic character.

Suitable plasticizers are for instance phthalic acid esters, e.g. dimethyl phthalate, dibutyl phthalate, diisononyl phthalate, and bis-(2-ethyl-hexyl)phthalate, phosphoric esters, e.g. triphenyl phosphate, tributyl phosphate, tricresyl phosphate, and tert-butyl-phenyl diphenyl phosphate, adipic acid derivatives, e.g. dibutyl adipate, benzyl adipate, etc., triacetin, camphor, and other plasticizers as known in the art.

The composition may also contain dyes, inorganic or organic colored pigments, pearly pigments, or

even fluorescent dyes (fluoresceins) as known in this field. Finally, the composition may also contain secondary film-forming agents, which favor adherence of the enamel or varnish film to the nail, thickening agents as well as other additives which are suitably employed in the preparation of conventional nail enamels or varnishes.

As for the amount of photochromic compound which must be present in said compositions to provide the desired photochromic effect, this may vary between 0.01 and 5 %. by weight, where the optimum concentration range for the preparation of a varnish, is preferably from 0.02 to 2 %, and more preferably from 0.05 and 1 %, and the optimum concentration range, for the preparation of a nail enamel, is preferably from 0.05 and 5 % and more preferably from 0.1 and 3 %.

In fact when the varnish or the enamel is originally colored, the new color tone which is obtained by exposing it to the light source, is the resultant of the combination of the original color with that provided by the activated form of the compound of formula (I); a higher concentration of photochromic compound is therefore needed in the preparation of nail enamels which generally are strongly colored, to obtain a noticeable photochromic effect.

As for the proportions between the other components, these are selected from the conventional ranges which are known to the person skilled in this art or can be easily set up by said skilled person on the basis of his personal knowledge, depending on the results which are desired.

In the following, some representative examples of nail enamels or varnishes according to the present invention are reported. These examples are only aimed at showing the photochromic effects of enamels and varnishes containing representative photochromic compounds of formula (I).

In the examples there are reported :

a) the varnish or enamel composition

b) the original color of the enamel or varnish film once dried on the nail

c) the color of the enamel or varnish film during exposure to direct sunlight at 5-25 °C (the best photochromic effect, and therefore the best color saturation, is in fact obtained when the temperature of the environment where exposure of the varnished nail occurs is lower than 25 °C. At higher temperatures the photochromic effect gradually diminishes)

d) the time needed for full activation

e) the time needed to get an 80-90 % extinction of color saturation produced by the photochromic effect (inactivation time)

f) the stability of color saturation and color tone upon exposure to morning sunlight at about 10 °C.

Example 1

Varnish composition

a)

| | |
|---|---|
| - CAB 381-0.1 (i.e. cellulose acetobutyrate manufactured by Eastman, (37 % butyryl, 13 % acetyl, 1.5 % hydroxyl, by weight) | 22 % w/w |
| - Ethyl acetate | 25 |
| - Butyl acetate | 25 |
| - Ethanol | 5 |
| - Toluene | 15 |
| - Dibutylphthalate | 7.5 |
| - 1,3,3-trimethyl-spiro[indoline-2,3′-[3H]naphtho[2,1-b][1,4]oxazine] ((I) : $R_1,R_2,R_6,R_7$ = H, $R_3,R_4,R_5$ = $-CH_3$, X = =CH-) | 0.5 |

b) Transparent and colorless

c) Greenish bleu

d) 10 seconds

e) 30 seconds

f) After 4 hour exposure, color saturation and color tone have not changed significantly with respect to the original ones.

## Example 2

Varnish composition

a)

| | |
|---|---|
| - CAB 381-0.1 | 17 % w/w |
| - Cellulose nitrate | 3 |
| - Ethyl acetate | 25 |
| - Butyl acetate | 25 |
| - Ethanol | 5 |
| - Toluene | 16 |
| - Dibutylphthalate | 8.5 |
| - 1,3,3,4,5-pentamethyl-spiro[indoline-2,3′-[3H]naphtho[2,1-b][1,4]oxazine] ((I) : $R_6$,$R_7$ = H, $R_1$,$R_2$,$R_3$,$R_4$,$R_5$ = $-CH_3$, X = =CH- | 0.5 |

b) Transparent and colorless
c) Greenish bleu
d) 10 seconds
e) 30 seconds
f) After 4 hour exposure, color saturation and color tone have not changed significantly with respect to the original ones.

## Example 3

Varnish composition

a)

| | |
|---|---|
| - CAB 381-0.1 | 17 % w/w |
| - Polymethacrylate | 5 |
| - Ethyl acetate | 25 |
| - Butyl acetate | 25 |
| - Ethanol | 5 |
| - Toluene | 15 |
| - Tricresylphosphate | 7.8 |
| - 1,3,3-trimethyl-spiro[indoline-6′-(1-piperidyl)-2,3′-[3H]naphtho[2,1-b][1,4]oxazine] ((I) : $R_1$,$R_2$,$R_7$ = H, $R_3$,$R_4$,$R_5$ = $-CH_3$ Rod1>¶ = 1-piperidyl, X = =CH-) | 0.2 |

b) Transparent and colorless
c) Violet
d) 10 seconds
e) 60 seconds

f) After 4 hour exposure, color saturation is about 80 % of the original one and color tone has not changed.

## Example 4

### Varnish composition

a)

| | |
|---|---|
| - CAB 381-0.1 | 22 % w/w |
| - Ethyl acetate | 24 |
| - Butyl acetate | 24 |
| - Ethanol | 4 |
| - Toluene | 13 |
| - Dibutylphthalate | 7.8 |
| - Pearl Essence (natural guanine extracted from fish scales) | 5 |
| - 1,3,3-trimethyl-spiro[indoline-6′-(1-piperidyl)-2,3′-[3H]naphtho[2,1-b][1,4]oxazine] ((I) : $R_1$,$R_2$,$R_7$ = H, $R_1$ = 1-piperidyl, $R_3$,$R_4$,R∫ = $-CH_3$, X = =CH-) | 0.2 |

b) Colorless, with a characteristic pearly appearance
c) Pearly violet
d) 10 seconds
e) 60 seconds
f) After 4 hour exposure, color saturation is still about 80 % of the original one and color tone has not changed.

## Example 5

### Enamel composition

a)

| | |
|---|---|
| - CAB 381-0.1 | 22 % w/w |
| - Ethyl acetate | 25 |
| - Butyl acetate | 25 |
| - Ethanol | 5 |
| - Toluene | 14 |
| - Dibutylphthalate | 7.4 |
| - $TiO_2$ | 1.05 |
| - D&C Red No.31 | 0.05 |
| - 1,3,3-trimethyl-spiro[indoline-2,3′-[3H]naphtho[2,1-b][1,4]oxazine] ((I) : $R_1$,$R_2$,$R_6$,$R_7$ = H, $R_3$,$R_4$,$R_5$ = $-CH_3$, X = =CH-) | 0.5 |

b) Light pink
c) Violet
d) 10 seconds
e) 30 seconds

f) After 4 hour exposure, color saturation and color tone have not changed significantly with respect to the original ones.

Comparative examples

Two representative spiro-benzopyran-indolines are formulated as in the foregoing examples, and more particularly as in example 1, and the obtained varnishes are compared with those of the present invention.

Example 6

Varnish composition

a)

| | |
|---|---|
| - CAB 381-0.1 | 22 % w/w |
| - Ethyl acetate | 25 |
| - Butyl acetate | 25 |
| - Ethanol | 5 |
| - Toluene | 15 |
| - Dibutylphthalate | 7.8 |
| - 1′,3′,3′-trimethyl-6-nitro-spiro-2H-1-benzopyran-2,2′-indoline | 0.2 |

The varnish film, when exposed to sunlight, changes its color to red-violet in 10 seconds.

The time needed to return to the colorless, inactivated form is very long : after one hour from discontinuing the exposure the film still has about 50 % of the color saturation noticed during exposure to sunlight.

Furthermore, upon continued exposure to sunlight, at 10 °C, the photochromic compound rapidly degradates.

In fact, after 30 minute exposure, a color saturation of about only 30 % of the original one can be noticed, and after 2 hours the photochromic effect is almost nil and the film is of an unpleasant irreversible yellowish color.

Example 7

Varnish composition

a)

| | |
|---|---|
| - CAB 381-0.1 | 22 % w/w |
| - Ethyl acetate | 25 |
| - Butyl acetate | 25 |
| - Ethanol | 5 |
| - Toluene | 15 |
| - Dibutylphthalate | 7.8 |
| - 1′,3′,3′-trimethyl-8-methoxy-6-nitro-spiro-2H-1-benzopyran-2,2′-indoline | 0.2 |

The film, originally of a bluish color, when exposed to sunlight, changes its color to a brilliant bleu in a

few seconds.

The time needed to return to the original color is very long : after one hour from discontinuing the exposure the film still has about 50 % of the color saturation noticed during exposure to sunlight.

Furthermore, upon continued exposure to sunlight, at 10° C, the photochromic compound rapidly degradates. In fact, after 30 minute exposure, a color saturation of about only 50 % of the original one can be noticed, and after 2 hours the photochromic effect is almost nil and the film is of a brown-violet color.

This last coloration is irreversible.

## Claims

1) A photochromic nail enamel or varnish composition comprising one or more film-forming agents, one or more organic solvents, and optionally, one or more plasticizers, devoid of acidic character, and a photochromically effective amount of at least one compound of general formula (I)

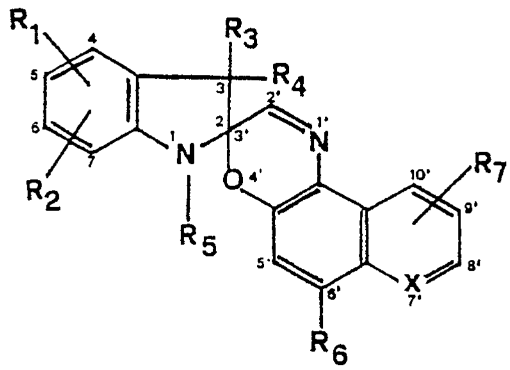

wherein

$R_1$ and $R_2$, which may be linked to any of positions 5, 5, 6, and 7, each independently, represent hydrogen, chloro, fluoro, bromo, straight or branched, optionally perfluorinated, $(C_1-C_5)$alkyl, straight or branched $(C_1-C_5)$alkoxy, nitro, or cyano;

$R_3$ and $R_4$, each independently, represent straight or branched $(C_1-C_5)$alkyl, phenyl or benzyl; or $R_3$ and $R_4$, taken together with the adjacent carbon atom, form a $(C_5-C_8)$cycloalkyl ring;

$R_5$ represents straight or branched $(C_1-C_5)$alkyl, phenyl, benzyl or allyl;

$R_6$ designates hydrogen, straight or branched $(C_1-C_5)$alkyl or $-NR_8R_9$, wherein $R_8$ is straight or branched $(C_1-C_5)$alkyl, phenyl, or benzyl, $R_9$ is hydrogen or has the same meaning as $R_8$, or $R_8$ and $R_9$ taken together with the adjacent nitrogen atom, form a 5-12 membered cyclic structure which may optionally contain an additional heteroatom selected from oxygen and nitrogen;

$R_7$, which may be linked to any of positions 7', 8', 9', or 10', represents hydrogen, fluoro, chloro, bromo, straight or branched $(C_1-C_5)$alkyl, straight or branched $(C_1-C_5)$alkoxy, cyano, thioether, or $(C_1-C_3)$-alkoxycarbonyl, or $R_7$ may represent a fused aromatic or heterocyclic ring, and

X is =CH- or =N-.

2) The nail enamel or varnish composition of claim 1 wherein

$R_1$ and $R_2$, each independently, represents hydrogen or methyl;

$R_3$ and $R_4$ are methyl or taken together with the adjacent carbon atom represent a cyclohexyl group;

$R_5$ is methyl;

$R_6$ represents hydrogen or $-NR_8R_9$, wherein $R_8$ and $R_9$ taken together with the adjacent nitrogen atom, form a 5-12 membered cyclic structure which may optionally contain an additional heteroatom selected from oxygen and nitrogen;

$R_7$ is hydrogen; and

X is =CH-.

3) The nail enamel or varnish composition of claim 2 wherein

$R_6$ represents hydrogen or $-NR_8R_9$, wherein $R_8$ and $R_9$ taken together with the adjacent nitrogen atom, form a piperidyl, morpholyl, pyrrolidyl, or hexamethyleneinmino group.

4) The nail enamel or varnish composition of claim 3 wherein the photochromic compound is selected from the group consisting of 1,3,3,4,5- or 1,3,3,5,6-pentamethyl-spiro[indoline-2,3′-[3H]-naphtho[2,1-b]-[1,4]-oxazine], 1,3,3-trimethyl-spiro[indoline-2,3′ -[3H]naphtho[2,1-b][1,4]oxazine], 1,3,3,4,5- or 1,3,3,5,6-pentamethyl-spiro[indoline-6′-(1-piperidyl)-2,3′-[3H]naphtho[2,1-b][1,4]oxazine], 1,3,3-trimethyl-spiro-[indoline-6′-(1-piperidyl)-2,3′-[3H]naphtho[2,1-b][1,4]oxazine], 1,3,3-trimethyl-spiro[indoline-6′-(1-morpholinyl)-2,3′-[3H]naphtho[2,1-b][1,4]oxazine], and 1,3,3-trimethyl-spiro[ indoline-6′-(1-piperidyl)-9′-methoxy-2,3′-[3H]naphtho[2,1-b][1,4]oxazine].

5) The nail enamel or varnish composition of claim 1 wherein the compound or compounds of formula (I) are present in an overall amount of from 0.01 to 5 % by weight.

6) The nail enamel or varnish composition of claim 1 which also comprises stable coloring agents.

7) The nail enamel or varnish composition of claim 1 wherein the film-forming agent or agents are selected from the group consisting of cellulose esters, polyacrylic and polymethacrylic esters, polyvynylacetate and polystyrene.

8) The nail enamel or varnish composition of claim 1 wherein the organic solvent or solvents are selected from the group consisting of lower alkanols, organic acetates, methylene-, ethylene-, and propylene-chlorides, toluene, and naphthene aromatics.

9) The nail enamel or varnish composition of claim 1 wherein the plasticizer or plasticizers are selected from the group consisting of phthalic acid esters, phosphoric esters, adipic acid derivatives, triacetin, and camphor.

10) A process for the preparation of a nail enamel or varnish composition according to claim 1, which comprises combining a phtochromically effective amount of at least one compound of formula (I)

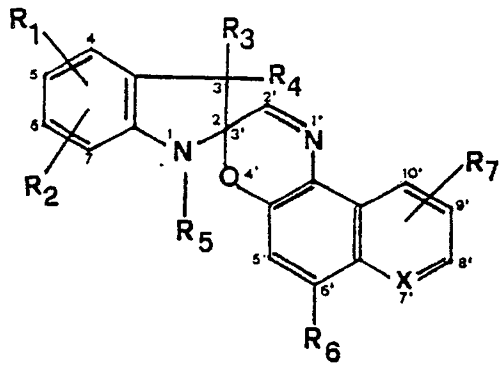

wherein $R_1$, $R_2$, $R_3$, $R_4$, $R_5$, $R_6$, $R_7$, and X are as defined in claim 1,
with one or more film-forming agents, one or more organic solvents, and, optionally, other conventional additives, devoid of acidic character.